(19)

Europäisches Patentamt
European Patent Office
Office européen des brevets

(11) **EP 2 910 171 A1**

(12) **EUROPEAN PATENT APPLICATION**
published in accordance with Art. 153(4) EPC

(43) Date of publication:
**26.08.2015 Bulletin 2015/35**

(51) Int Cl.:
***A61B 1/00*** *(2006.01)* ***G02B 23/24*** *(2006.01)*

(21) Application number: **13846290.8**

(86) International application number:
**PCT/JP2013/077680**

(22) Date of filing: **10.10.2013**

(87) International publication number:
**WO 2014/061566 (24.04.2014 Gazette 2014/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.10.2012 JP 2012229255**

(71) Applicant: **Olympus Corporation**
**Shibuya-ku**
**Tokyo 151-0072 (JP)**

(72) Inventors:
• **TOJO, Ryo**
  **Shibuya-ku**
  **Tokyo 151-0072 (JP)**

• **HANE, Jun**
  **Shibuya-ku**
  **Tokyo 151-0072 (JP)**
• **FUJITA, Hiromasa**
  **Shibuya-ku**
  **Tokyo 151-0072 (JP)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(54) **OBSERVATION APPARATUS, OBSERVATION ASSISTANCE DEVICE, OBSERVATION ASSISTANCE METHOD AND PROGRAM**

(57)    A relative position information acquiring section (61) acquires relative position information, in relation to an insertion subject (2), of a portion of an inserting section (31) which becomes a position detection object, on the basis of displacement amount information of the inserting section, that is inserted into the insertion subject, from a fiber shape sensor (4) and an insertion and rotation detecting section (5). An image acquisition position calculating section (63) calculates an image acquisition position (P) that is at least one of an image acquisition region (83) as a region of the insertion subject which is being acquired image by an image acquisition section (34), a part (84) of the image acquisition region and a point in the image acquisition region, by use of the relative position and shape information of the insertion subject acquired by an insertion subject shape acquiring section (62). A display calculating section (64) calculates weighting information of the image acquisition position (P) on the basis of a weighting index parameter, and sets a display format on the basis of the weighting information. An output section (65) outputs the display format and the image acquisition position (P) as display information.

EP 2 910 171 A1

Insertion subject
shape information

Insertion subject
shape
acquiring section — 62

Image acquisition
position
calculating section — 63

Relative position
information
acquiring section — 61

Display calculating
section — 64

Output section — 65

Observation
supporting device

F I G. 1A

**Description**

Technical Field

The present invention relates to an observation apparatus in which an inserting section is inserted into an insertion subject for observation, an observation supporting device for use in such an observation apparatus, an observation supporting method, and a program which allows a computer to execute a procedure of the observation supporting device.

Background Art

As a supporting device in a case where an inserting section is inserted into an insertion subject for observation, for example, there is disclosed, in U.S. Patent No. 6,846,286, a constitution to display a shape of an endoscope inserting section in a display section when the endoscope inserting section is inserted into a human body.

As to this constitution, in an endoscope device, flexible bend detecting optical fibers having bend detecting portions in which a quantity of light to be transmitted changes in accordance with a size of an angle of a bend are attached to a flexible band-like member in a state where the fibers are arranged in parallel, and the band-like member is inserted into and disposed in the endoscope inserting section along a substantially total length of the endoscope inserting section. Additionally, a bending state of the band-like member in a portion where each bend detecting portion is positioned is detected from the light transmission quantity of each bend detecting optical fiber, to display the bending state as the bending state of the endoscope inserting section in a monitor screen.

In general, there are only a few regions that become marks in an insertion subject, and hence when it is not easily judged only from an acquired image which region of the insertion subject is being observed, it is also not easily judged whether or not all required regions could be imaged (observed).

U.S. Patent No. 6,846,286 mentioned above discloses that a shape of an inserting section is detected and displayed. However, there has not been suggested a method of detecting and displaying which region of the insertion subject is being imaged (observed).

Summary of Invention

The present invention has been developed in respect of the above, and an object thereof is to provide an observation apparatus, an observation supporting device, an observation supporting method and a program that can supply, to an operator, information to judge which region of an insertion subject is being imaged.

According to a first aspect of the invention, there is provided an observation apparatus characterized by comprising an inserting section that is to be inserted into an insertion subject and includes an image acquisition opening, an image acquisition section that receives light entering into the image acquisition opening and acquires image, a relative position detecting section that detects a relative position, in relation to the insertion subject, of a portion of the inserting section which becomes a position detection object, an insertion subject shape acquiring section that acquires shape information of the insertion subject, an image acquisition position calculating section that calculates an image acquisition position that is at least one of an image acquisition region as a region of the insertion subject which is being acquired image by the image acquisition section, a part of the image acquisition region and a point in the image acquisition region, by use of the relative position and the shape information of the insertion subject, a display calculating section that calculates weighting information of the image acquisition position on the basis of a weighting index parameter, and sets a display format on the basis of the weighting information, and an output section that outputs the display format and the image acquisition position as display information.

According to a second aspect of the invention, there is provided an observation supporting device for use in an observation apparatus in which an inserting section is inserted into an insertion subject to acquire image of the inside of the insertion subject, the observation supporting device characterized by comprising a relative position information acquiring section that acquires relative position information, in relation to the insertion subject, of a portion of the inserting section which becomes a position detection object, on the basis of displacement amount information of the inserting section, an insertion subject shape acquiring section that acquires shape information of the insertion subject, an image acquisition position calculating section that calculates an image acquisition position that is at least one of an image acquisition region as a region of the insertion subject which is being acquired image by the observation apparatus, a part of the image acquisition region and a point in the image acquisition region, by use of the relative position information and the shape information of the insertion subject, a display calculating section that calculates weighting information of the image acquisition position on the basis of a weighting index parameter, and sets a display format on the basis of the weighting information, and an output section that outputs the display format and the image acquisition position as display information.

According to a third aspect of the invention, there is provided an observation supporting method for use in an observation apparatus in which an inserting section is inserted into an insertion subject to acquire image of the inside of the insertion subject, the observation supporting method characterized by comprising a position information acquiring step of acquiring relative position information, in relation to the insertion subject, of a position of the inserting section which becomes a detection object, on the basis of displacement amount information of the inserting section, an insertion subject shape acquiring step of acquiring shape information of the insertion subject, an image acquisition position calculating step of calculating an image acquisition position that is at least one of an image acquisition region as a region of the insertion subject which is being acquired image by the observation apparatus, a part of the image acquisition region and a point in the image acquisition region, by use of the relative position information and the shape information of the insertion subject, a display calculating step of calculating weighting information of the image acquisition position on the basis of a weighting index parameter, and setting a display format on the basis of the weighting information, and an output step of outputting the display format and the image acquisition position as display information.

According to a forth aspect of the invention, there is provided a program which allows a computer to execute a position information acquiring procedure of acquiring relative position information, in relation to an insertion subject, of a position of an inserting section which becomes a detection object, on the basis of displacement amount information of the inserting section in an observation apparatus in which the inserting section is inserted into the insertion subject to acquire image of the inside of the insertion subject, an insertion subject shape acquiring procedure of acquiring shape information of the insertion subject, an image acquisition position calculating procedure of calculating an image acquisition position that is at least one of an image acquisition region as a region of the insertion subject which is being acquired image by the observation apparatus, a part of the image acquisition region and a point in the image acquisition region, by use of the relative position information and the shape information of the insertion subject, a display calculating procedure of calculating weighting information of the image acquisition position on the basis of a weighting index parameter, and setting a display format on the basis of the weighting information, and an output procedure of outputting the display format and the image acquisition position as display information.

According to the present invention, it is possible to supply information to judge which region of an insertion subject is being imaged, and hence an operator can easily judge which region of the insertion subject is being imaged and whether or not all required regions could be imaged. Therefore, it is possible to provide an observation apparatus, an observation supporting device, an observation supporting method and a program which can prevent oversight of observation regions.

Furthermore, according to the present invention, it is possible to display an image acquisition position in a display format based on weighting information of the image acquisition position, and hence it is possible for an operator to easily judge an importance of the image acquisition position.

Brief Description of Drawings

FIG. 1A is a view showing a schematic constitution of an observation supporting device according to a first embodiment of the present invention and an observation apparatus to which the device is applied;

FIG. 1B is a view for explaining an example where information is supplied via a display device connected to the observation apparatus according to the first embodiment;

FIG. 2A is a view showing a case where a bending portion is bent in an upward direction of the paper surface to explain a principle of a fiber shape sensor;

FIG. 2B is a view showing a case where the bending portion is not bent to explain the principle of the fiber shape sensor;

FIG. 2C is a view showing a case where the bending portion is bent in a downward direction of the paper surface to explain the principle of the fiber shape sensor;

FIG. 3 is a view showing an attaching structure by which the fiber shape sensor is attached to an inserting section;

FIG. 4A is a view for explaining a constitution of an insertion and rotation detecting section;

FIG. 4B is a view for explaining an operation principle of the insertion and rotation detecting section;

FIG. 5A is a view showing an operation flowchart of the observation supporting device according to the first embodiment in a case where a speed of an image acquisition position is used as a weighting index parameter;

FIG. 5B is a diagram showing a relation between the speed of the image acquisition position and weighting information in a case where the speed of the image acquisition position as one example of the weighting index parameter is used;

FIG. 6A is a view for explaining which position of the insertion subject is to be displayed by a first position display;

FIG. 6B is a view for explaining which position of the insertion subject is to be displayed by a second position display;

FIG. 7A is a diagram showing a relation between the weighting information and a display color of the image acquisition position;

FIG. 7B is a diagram showing a display example in a case where the display color of the image acquisition position is changed on the basis of the weighting information;

FIG. 8A is a view for explaining use of a distance between an image acquisition opening and the image acquisition position as another example of the weighting index parameter;

FIG. 8B is a diagram showing a relation of the distance between the image acquisition opening and the image acquisition position to the weighting information;

FIG. 9A is a view for explaining another weighting technique concerning the distance between the image acquisition opening and the image acquisition position;

FIG. 9B is a diagram showing a relation between a focusing distance and the weighting information;

FIG. 10A is a view for explaining use of an image acquisition angle formed by an image acquisition direction that is a direction from the image acquisition opening to a center of an image acquisition range and a plane of the image acquisition position, as another example of the weighting index parameter;

FIG. 10B is a diagram showing a relation between the image acquisition angle and the weighting information;

FIG. 11 is a diagram showing a relation between a stop time of the image acquisition position and the weighting information in a case where the stop time of the image acquisition position as a further example of the weighting index parameter is used;

FIG. 12A is a view for explaining use of a temporal change of a bend amount that is an angle formed by one longitudinal direction of the inserting section and the other longitudinal direction of the inserting section via the bending portion, as a further example of the weighting index parameter;

FIG. 12B is a diagram showing a relation between the bend amount and the weighting information;

FIG. 13A is a view for explaining use of a brightness of an image acquired by an image acquisition section as a further example of the weighting index parameter;

FIG. 13B is a diagram showing a relation between the number of pixels of halation + black defects and the weighting information;

FIG. 14 is a view for explaining a blurring amount of the image in a case where the blurring amount of the image is used as a further example of the weighting index parameter;

FIG. 15 is a view for explaining a predetermined range in which the weighting information is calculated concerning the weighting index parameter set on the basis of the image acquired by the image acquisition section;

FIG. 16 is a view for explaining a change of a density of points as a further example of a display format set on the basis of the weighting information;

FIG. 17 is a view showing an operation flowchart of the observation supporting device according to the first embodiment in a case where the weighting information is calculated by using the weighting index parameters;

FIG. 18A is a view for explaining a still further example of the display format set on the basis of the weighting information;

FIG. 18B is a view for explaining another display example;

FIG. 18C is a view for explaining still another display example;

FIG. 19A is a view showing a state before rotation to explain a change of an acquired image due to the rotation of the inserting section;

FIG. 19B is a view showing a state after the rotation;

FIG. 20A is a view showing an operation flowchart of an observation supporting device according to a second embodiment of the present invention;

FIG. 20B is a diagram showing a relation among a speed of an image acquisition position, a threshold value of the speed and weighting information to explain a technique of comparing a weighting index parameter with the threshold value to calculate the weighting information;

FIG. 20C is a view for explaining an example of a display format in which a locus display of the image acquisition position having a small weight is not performed; and

FIG. 21 is a view showing an operation flowchart of the observation supporting device according to the second embodiment in a case where the weighting information is calculated by using the weighting index parameters.

Description of Embodiments

Hereinafter, a mode for carrying out the present invention will be described with reference to the drawings.

[First Embodiment]

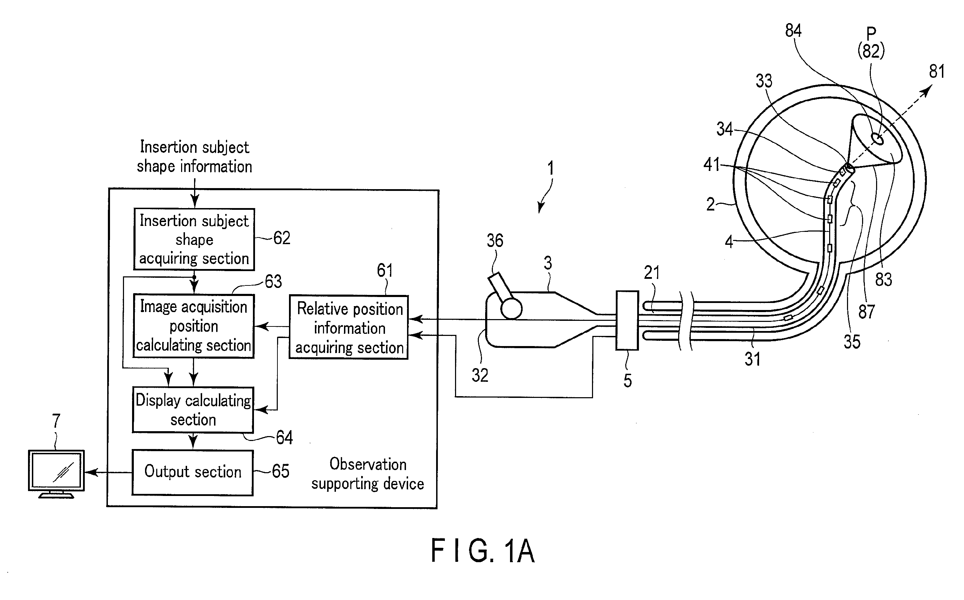

As shown in FIG. 1A, an observation apparatus 1 concerned with a first embodiment of the present invention includes an inserting tool 3 including an inserting section 31 to be inserted into an insertion subject 2. The observation apparatus 1 further includes a fiber shape sensor 4 and an insertion and rotation detecting section 5 as detecting sections to detect displacement amount information of the inserting section 31. The observation apparatus 1 further includes an observation supporting device 6 concerned with the first embodiment of the present invention which calculates display information to support observation on the basis of shape information of the insertion subject 2 and the displacement amount information of the inserting section 31. The observation apparatus 1 also includes a display device 7 that displays the display information.

The inserting tool 3 is, for example, an endoscope device. The inserting tool 3 includes the inserting section 31 and an operating section 32 constituted integrally with the inserting section 31.

The inserting section 31 is a flexible tubular member and is insertable from an insertion port 21 of the insertion subject 2 into the insertion subject 2. In an end portion of the inserting section 31 in an inserting direction (hereinafter referred to as an inserting section distal end), an image acquisition opening 33 is disposed. Further, in the vicinity of the inserting section distal end in the inserting section 31, an image acquisition section 34 is included. The image acquisition section 34 receives light entering into the image acquisition opening 33 to acquire image. An image acquired by the image acquisition section 34 is output to the display device 7 through the observation supporting device 6.

It is to be noted that needless to say, the image acquisition section 34 may not be disposed in the vicinity of the inserting section distal end in the inserting section 31 but may be disposed in the operating section 32. In this case, the image acquisition section 34 is connected to the image acquisition opening 33 by a light guide or the like to guide the light entering into the image acquisition opening 33 to the image acquisition section 34.

In addition, the inserting section 31 includes a bending portion 35 in the vicinity of the inserting section distal end. The bending portion 35 is coupled with an operation lever 36 disposed in the operating section 32 by a wire, though not especially shown in the drawing. In consequence, the operation lever 36 is moved to pull the wire, thereby enabling a bending operation of the bending portion 35.

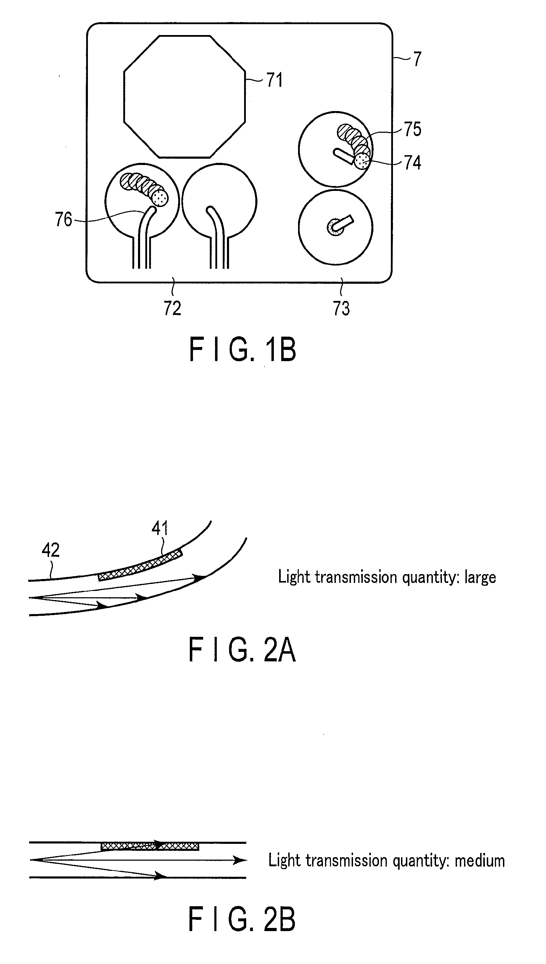

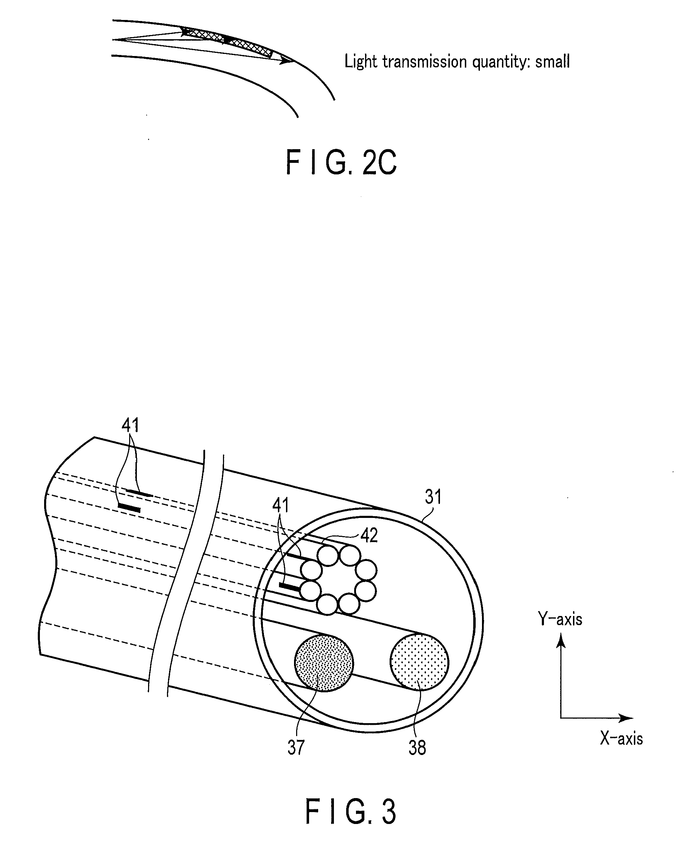

In addition, the fiber shape sensor 4 is disposed in the inserting section 31. The fiber shape sensor 4 includes optical fibers. Each optical fiber is provided with a bend detecting portion 41 in one portion thereof. In the bend detecting portion 41, a clad of the optical fiber is removed to expose a core thereof, and a light absorbing material is applied to the core to constitute the bend detecting portion. In the bend detecting portion 41, as shown in FIG. 2A to FIG. 2C, a quantity of light to be absorbed by the bend detecting portion 41 changes in accordance with a bend of the bending portion 35. Therefore, a quantity of the light to be guided in an optical fiber 42 changes, i.e., a light transmission quantity changes.

In the fiber shape sensor 4 of this constitution, for the purpose of detecting the bend in an X-axis direction and the bend in a Y-axis direction shown in FIG. 3, two optical fibers 42 are disposed so that the two bend detecting portions 41 directed in the X-axis direction and the Y-axis direction, respectively, form a pair, to detect a bend amount of one region. Furthermore, the optical fibers 42 are disposed so that the pair of bend detecting portions 41 are arranged in a longitudinal direction (an inserting direction) of the inserting section 31. Furthermore, light from an unshown light source is guided by each of the optical fibers 42, and the light transmission quantity that changes in accordance with the bend amount of each of the optical fibers 42 is detected by an unshown light receiving section. The thus detected light transmission quantity is output as one piece of the displacement amount information of the inserting section 31 to the observation supporting device 6.

It is to be noted that a portion other than the bending portion 35 of the inserting section 31 freely bends in accordance with an internal structure of the insertion subject 2 due to a flexibility of the inserting section 31. Therefore, the bend detecting portions 41 are preferably disposed not only in the bending portion 35 of the inserting section 31 but also on an operating section side from the bending portion, so that it is possible to also detect a bending state of the portion other than the bending portion 35 of the inserting section 31.

It is to be noted that as shown in FIG. 3, an illuminating optical fiber 37 and a wiring line 38 for the image acquisition section are also disposed in the inserting section 31. The light from the unshown illuminating light source disposed in the operating section 32 is guided by the illuminating optical fiber 37, and emitted as illuminating light from the inserting section distal end. The image acquisition section 34 can acquire image of the inside of the insertion subject 2 that is a dark part by this illuminating light.

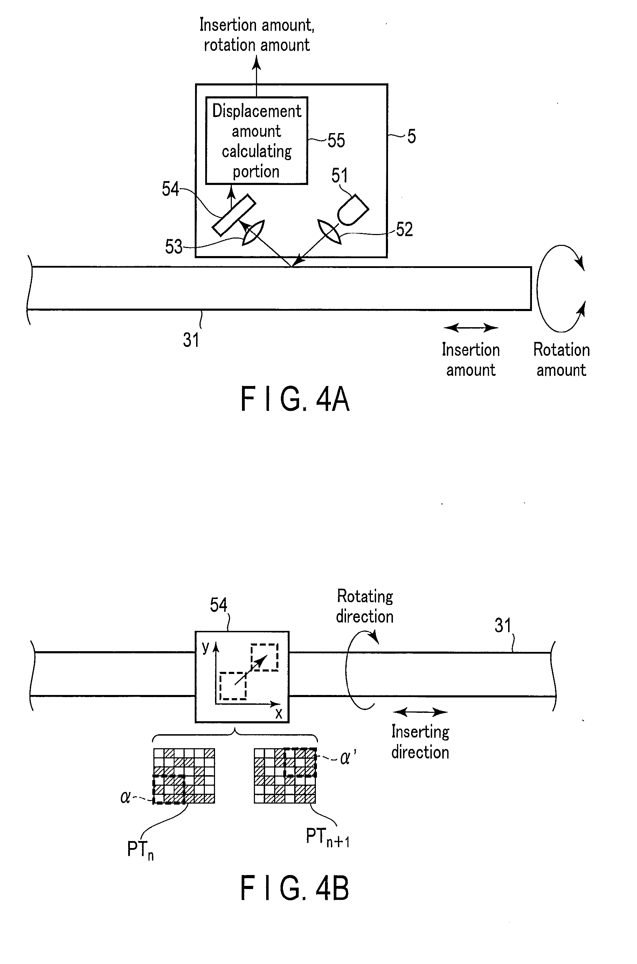

In addition, as shown in FIG. 1A, the insertion and rotation detecting section 5 is disposed in the vicinity of the insertion port 21 of the insertion subject 2. The insertion and rotation detecting section 5 detects an insertion amount and a rotation amount of the inserting section 31 to output the amounts as one piece of the displacement amount information of the inserting section 31 to the observation supporting device 6. Specifically, as shown in FIG. 4A, the insertion and rotation detecting section 5 is constituted of a light source 51, a projection lens 52, a light receiving lens 53, an optical pattern detecting portion 54, and a displacement amount calculating portion 55.

The inserting section 31 is irradiated with the light emitted from the light source 51 through the projection lens 52. The light reflected by the inserting section 31 is received through the light receiving lens 53 by the optical pattern detecting portion 54. The optical pattern detecting portion 54 detects images of a plane of the inserting section 31 which is an optical pattern continuously at detection times $t_0$, $t_1$, $t_2$, ..., $t_n$, ....

The displacement amount calculating portion 55 calculates a displacement amount by use of the optical patterns present in the images of two pieces of image data acquired by the optical pattern detecting portion 54 at different times.

More specifically, as shown in FIG. 4B, one optical pattern is any selected reference pattern $\alpha$ present in the image (an optical pattern $PT_n$) of the image data acquired at any time $t_n$. The other optical pattern is an optical pattern $\alpha'$ that is present in a part of the image (an optical pattern $PT_{n+1}$) of the image data acquired at any time $t_{n+1}$ after the elapse of time from $t_n$ and that matches the above reference pattern $\alpha$. The displacement amount calculating portion 55 compares a displacement of the reference pattern $\alpha$ on the image data with that of the optical pattern $\alpha'$ on the image data, and calculates the displacement amount on the image in each of an x-axis direction and a y-axis direction. Here, as shown in FIG. 4B, the optical pattern detecting portion 54 is positioned so that an x-axis of the optical pattern detecting portion 54 matches an axial direction of the inserting section 31. Therefore, a displacement amount $\Delta x_f$ in the x-axis direction which is calculated by the displacement amount calculating portion 55 is proportional to the insertion amount of the inserting section 31, and a displacement amount $\Delta y_f$ in the y-axis direction is proportional to the rotation amount of the inserting section 31. The insertion amount and the rotation amount in the images which are calculated by the displacement amount calculating portion 55 are output as the displacement amount information to the observation supporting device 6. It is to be noted that an increase/decrease direction of each displacement amount indicates directions of insertion and rotation of the inserting section 31, and hence the displacement amount information also includes information of the inserting direction and the rotating direction.

In addition, as shown in FIG. 1A, the observation supporting device 6 concerned with the present embodiment is constituted of a relative position information acquiring section 61, an insertion subject shape acquiring section 62, an image acquisition position calculating section 63, a display calculating section 64, and an output section 65.

The relative position information acquiring section 61 acquires relative position information, in relation to the insertion subject 2, of a portion of the inserting section 31 which becomes a position detection object, on the basis of the displacement amount information of the inserting section 31 which is input from the fiber shape sensor 4 and the insertion and rotation detecting section 5. That is, the relative position information acquiring section 61 cooperates with the fiber shape sensor 4 and the insertion and rotation detecting section 5 to function as a relative position detecting section that detects a relative position, in relation to the insertion subject 2, of the portion of the inserting section 31 which becomes the position detection object. The insertion subject shape acquiring section 62 acquires the shape information of the insertion subject 2.

The image acquisition position calculating section 63 calculates an image acquisition position that is at least one of an image acquisition region as a region of the insertion subject 2 being acquired image by the image acquisition section 34, a part of the image acquisition region and a point in the image acquisition region, by use of the above relative position and the above shape information of the insertion subject 2. The display calculating section 64 calculates weighting information of the image acquisition position on the basis of a weighting index parameter, and sets a display format on the basis of the weighting information. Furthermore, the output section 65 outputs this display format and the above image acquisition position as the display information. The display information output from the observation supporting device 6 is displayed by the display device 7.

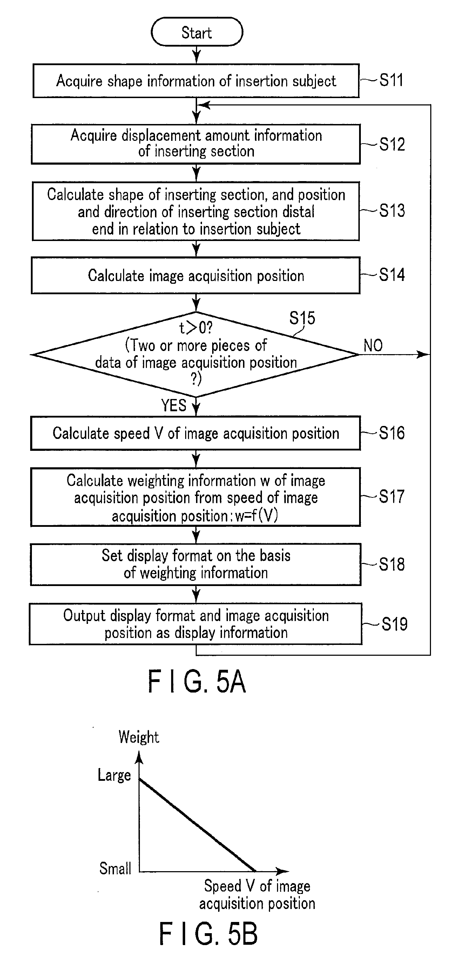

Hereinafter, an operation of the observation supporting device 6 will be described in detail with reference to an operation flowchart of FIG. 5A.

First, the insertion subject shape acquiring section 62 acquires the shape information (insertion subject shape information) including position information of a range of the insertion subject 2 which becomes an image acquisition object in relation to the insertion subject 2 (step S11). For example, this insertion subject shape information is constituted on the basis of data from the outside or inside of the insertion subject 2 before the inserting section 31 is inserted into the insertion subject 2.

That is, the insertion subject shape information based on the data from the outside is constituted by utilizing an apparatus that can detect the information by use of the light transmitted through the insertion subject 2, for example, a CT diagnosis apparatus, an ultrasonic diagnosis apparatus or an X-ray apparatus.

In addition, the insertion subject shape information based on the data from the inside is constituted by utilizing locus data obtained when the inserting section 31 is moved in a space of the insertion subject 2 or by connecting position information obtained when the inserting section distal end comes in contact with the insertion subject 2. When the position information obtained during the contact between the inserting section distal end and the insertion subject 2 is utilized, a size of the space can be detected, and the insertion subject shape information can more exactly be acquired. Furthermore, when the insertion subject 2 is a human organ, the information may be constituted by presuming a physical constitution, and when the insertion subject 2 is a structure, the information may be constituted by inputting the shape through a drawing.

It is to be noted that when the insertion subject shape information is acquired by the insertion subject shape acquiring section 62, the insertion subject shape information may directly be acquired from an apparatus such as the CT diagnosis apparatus by connecting the apparatus that constitutes the insertion subject shape information, or the insertion subject shape information may be acquired by storing the insertion subject shape information output from the apparatus once in a storage medium and reading the stored insertion subject shape information or by downloading the insertion subject shape information via a network. Furthermore, the insertion subject shape acquiring section 62 is not limited to that interface or data reader and the acquiring section itself may be the apparatus that constitutes the insertion subject shape information.

The insertion subject shape information acquired by the insertion subject shape acquiring section 62 is output to the image acquisition position calculating section 63 and the display calculating section 64.

In addition, the relative position information acquiring section 61 acquires the displacement amount information of the inserting section 31 (step S12), and acquires a shape of the inserting section 31 and a position and a direction of the inserting section distal end to the insertion subject 2 (step S13).

Specifically, the relative position information acquiring section 61 includes a function of obtaining the shape of the inserting section 31, a function of obtaining the insertion amount and the rotation amount of the inserting section 31, and a function of obtaining the position and direction of the inserting section distal end in relation to the insertion subject 2.

That is, such a relational equation between a change $\Delta Q$ of the light transmission quantity of the fiber shape sensor 4 and a bend amount $\phi$ of the bend detecting portion 41 as in the following equation (1) is beforehand obtained and stored in the relative position information acquiring section 61.

$$\phi = f(\Delta Q) \qquad\qquad (1)$$

Furthermore, the relative position information acquiring section 61 calculates the bend amount of each bend detecting portion 41 from the light transmission quantity given as the displacement amount information from the fiber shape sensor 4 in accordance with this stored equation (1). Furthermore, the shape of the inserting section 31 is obtained from the bend amount of each bend detecting portion 41 and an arrangement interval of the respective bend detecting portions 41 which is given as foresight information.

In addition, coefficients $\underline{a}$ and b to convert the displacement amount on the image which is calculated by the displacement amount calculating portion 55 into an actual insertion amount and an actual rotation amount of the inserting section 31 are beforehand obtained and stored in the relative position information acquiring section 61. Furthermore, the relative position information acquiring section 61 multiplies the displacement amount on the image which is calculated by the displacement amount calculating portion 55 by the stored coefficients $\underline{a}$ and b as in the following equation (2) to calculate an insertion amount m and a rotation amount $\theta$.

$$m = a \times \Delta x$$

$$\theta = b \times \Delta y \qquad\qquad (2)$$

Afterward, the relative position information acquiring section 61 calculates the shape of the inserting section 31 in relation to the insertion subject 2 from the calculated shape of the inserting section 31 and the calculated insertion amount and rotation amount of the inserting section 31 in relation to the insertion subject 2. Furthermore, the section 61 calculates the relative position information, in relation to the insertion subject 2, of the portion of the inserting section 31 which becomes the position detection object, i.e., the position and direction of the inserting section distal end in relation to the insertion subject 2 (the position of the image acquisition opening 33 and a direction opposite to an incident direction of the light) from the shape of the inserting section 31 in relation to the insertion subject 2. The relative position information in relation to the insertion subject 2 which is obtained in this manner is output to the image acquisition position calculating section 63. In addition, shape information indicating the shape of the inserting section 31 in relation to the insertion subject 2 and information of an inserting section distal position in the above relative position information are output to the display calculating section 64.

Furthermore, the image acquisition position calculating section 63 calculates the image acquisition position from the relative position information obtained by the relative position information acquiring section 61 and the insertion subject shape information acquired by the insertion subject shape acquiring section 62 (step S14).

Specifically, for example, as shown in FIG. 1A, the image acquisition position calculating section 63 obtains an intersection 82 between a straight line including the position and direction of the inserting section distal end indicated by the relative position information (an image acquisition direction 81) and a shape of the insertion subject 2, i.e., a center of a viewing field (an image acquisition region 83), as an image acquisition position P.

In general, a region of interest in an observation object is at the center of the viewing field, and hence the center of the viewing field is often more important than a periphery thereof. It is to be noted that here, the description has been given as to the example where the intersection is obtained as the image acquisition position P, but the viewing field (the image acquisition region 83) that is the region of the insertion subject 2 being acquired image by the image acquisition section 34 may be calculated as the image acquisition position P. In consequence, a range in which image is acquired by the image acquisition section 34 can be grasped. In addition, a partial region 84 or a point in the viewing field (the image acquisition region 83) may be calculated as the image acquisition position P. For example, when the image acquisition region 83 cannot exactly be detected, a small region is calculated in consideration of an error, so that a region that is not imaged can be prevented from being wrongly detected as the imaged region. That is, an omission of observation can be prevented.

Image acquisition position information indicating the thus obtained image acquisition position P is output to the display calculating section 64.

Afterward, the display calculating section 64 calculates the weighting information of the image acquisition position P on the basis of the weighting index parameter, and executes an operation of setting the display format on the basis of the weighting information. Here, a case where a speed of the image acquisition position is used as the weighting index parameter is described as an example.

The display calculating section 64 first judges whether or not t is larger than 0, i.e., whether or not two or more pieces of data of the image acquisition position P are present (step S15). Here, when t is not larger than 0 (one piece of information of the image acquisition position P is only present), the step returns to the above step S12 to repeat the abovementioned operation. That is, immediately after the processing of the operation of the observation apparatus 1, only one piece of information of the image acquisition position P is obtained, and in this case, the speed cannot be calculated, and hence the shape of the inserting section 31 and the image acquisition position P are calculated again.

When two or more pieces of information of the image acquisition position are present, the display calculating section 64 performs the calculation of a speed V of the image acquisition position (step S16). That is, the display calculating section 64 obtains a speed $V_n$ of the image acquisition position P, which is the weighting index parameter, from the image acquisition position P at the current time t and an image acquisition position $P_{n-1}$ obtained at one previous image acquisition time $t_{n-1}$ in accordance with the following equation (3):

$$V_n = (P_n - P_{n-1}) / (t_n - t_{n-1}) \qquad (3)$$

Furthermore, the display calculating section 64 calculates weighting information w of the image acquisition position from this obtained speed V (step S17). For example, as shown in FIG. 5B, weighting is performed in accordance with a relation (the following equation (4)) in which the weighting information becomes smaller in proportion to the speed V of the image acquisition position.

$$w = f(V) \qquad (4)$$

That is, when the moving speed of the image acquisition position is fast, it is judged that an operator cannot perform the observation or that the operator is not performing the observation but is just moving the inserting section distal end, and the weighting information is made smaller. Conversely, when the moving speed of the image acquisition position is slow, it is judged that the operator can perform the observation, and the weighting information is enlarged.

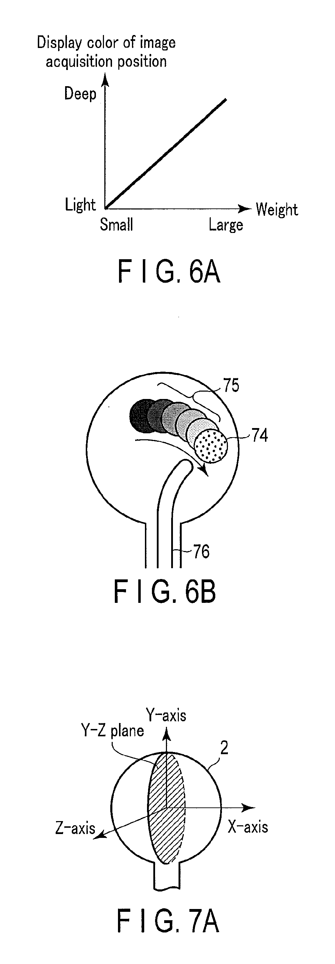

Furthermore, the display calculating section 64 sets the display format on the basis of this weighting information (step S18). That is, the display calculating section 64 holds the current image acquisition position P and locus information of the image acquisition position which is a past image acquisition position into an unshown internal memory or the like. Furthermore, the current image acquisition position and a locus of the image acquisition position are set so as to change the display format on the basis of the weighting information of the image acquisition position. For example, as shown in FIG. 6A, they are set so that a display color of the image acquisition position deepens in proportion to the weighting information.

Furthermore, the output section 65 outputs at least the above display format and the above image acquisition position (the current image acquisition position and the locus of the image acquisition position) as the display information (step S19). Afterward, the processing returns to the above step S12 to repeat the above operation.

The above display information can further include the shape information indicating the shape of the inserting section 31 in relation to the insertion subject 2, the insertion subject shape information, the image acquired by the image acquisition section 34 and the like. That is, as shown in FIG. 1B, the output section 65 prepares and outputs such display information as to display the image acquired by the image acquisition section 34 (an acquired image display 71) and two-dimensional views 72 and 73 obtained by dividing the insertion subject 2 as the insertion subject shape information by a predetermined region in the display device 7.

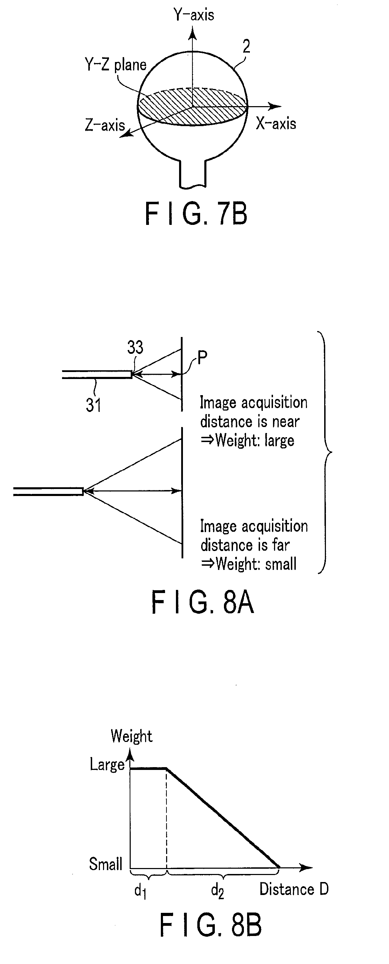

Here, the first two-dimensional view 72 is a view showing a state where the shape of the insertion subject 2 is divided by a Y-Z plane and opened in a right-left direction in a coordinate of the insertion subject 2 as shown in FIG. 7A. In addition, the second two-dimensional view 73 is a view having a view point different from that of the first two-dimensional view 72 and showing a state where the shape of the insertion subject 2 is divided by an X-Z plane and opened in an upward-downward direction in the coordinate of the insertion subject 2 as shown in FIG. 7B.

Furthermore, there is prepared such display information as to display a current position display 74 showing the current image acquisition position, a position locus display 75 showing the locus of the image acquisition position and an inserting section shape schematic display 76 showing the shape of the inserting section 31, on these two-dimensional views 72 and 73.

In addition, as shown in FIG. 6B, such display information as to achieve a certain identification display is preferably prepared by, for example, changing mutual colors or patterns or performing the position locus display 75 as a blinking display so that the current position display 74 and the position locus display 75 can be distinguished. Furthermore, a depth of the color of each of the current position display 74 and the position locus display 75 is displayed in the depth proportional to a weight which is a result calculated by the display calculating section 64. It is to be noted that the operator may be allowed to select the presence/absence of the identification display or a configuration of the identification display to distinguish the current position display 74 and the position locus display 75.

As described above, according to the present first embodiment, the observation supporting device 6 calculates the image acquisition position and the weighting information of the image acquisition position, and sets the display format of the image acquisition position on the basis of the weighting information of the image acquisition position, to output the display format and the image acquisition position as the display information. Therefore, it is possible for the operator to easily judge which region of the insertion subject 2 is being acquired image and whether images of all required regions can be acquired, and oversight of image acquisition regions can be prevented. Furthermore, importance of the image acquisition position can easily be judged by the operator.

In addition, the observation supporting device 6 detects the shape of the inserting section 31 with the fiber shape sensor 4, and detects the insertion amount and rotation amount of the inserting section 31 with the insertion and rotation detecting section 5. Therefore, the shape of the inserting section 31 in relation to the insertion subject 2 and a position and a direction of the image acquisition opening 33 can be detected.

It is to be noted that the fiber shape sensor 4 the insertion and rotation detecting section 5 optically detects the shape of the inserting section 31 inserted into the insertion subject 2 and a position and a direction of the image acquisition opening 33 as described above, but may detect the same by another method. For example, a coil is disposed in the vicinity of at least the image acquisition opening 33 in the inserting section 31 and a current is passed through the coil to generate a magnetic field which is received on the outside, or a magnetic field distribution generated on the outside is received by the coil, so that the position or direction of the coil, i.e., the image acquisition opening 33 can be detected. It is to be noted that when the coils are disposed in a longitudinal direction of the inserting section 31, the shape of the inserting section 31 can also be detected.

Furthermore, the weighting information is calculated in proportion to the speed of the image acquisition position. When the speed is fast, the weighting information is made smaller, and when the speed is slow, the weighting information is enlarged, so that it can be judged that the image acquisition position where the weighting information is small moves fast and therefore cannot be observed.

In addition, the current position display 74 showing the image acquisition position and the position locus display 75 showing the locus of the image acquisition position can be changed and displayed on the basis of the weighting information of the image acquisition position. For example, when the weighting information is large, i.e., the speed of the image acquisition position is slow, the display colors of the current position display 74 and the position locus display 75 can be changed so as to deepen. In addition, when the weighting information is small, i.e., the speed of the image acquisition position is fast, the display colors of the current position display 74 and the position locus display 75 can be changed so as to lighten. When the display is changed in this manner, the importance of the information of the image acquisition position is visually easily recognized.

Additionally, it has been described that the weighting information is proportional to the speed of the image acquisition position, but the weighting information may be represented by another relational equation such as an exponential function.

In addition, it has been described that the weighting index parameter is the speed of the image acquisition position, but the parameter may be the speed of the inserting section distal end, i.e., the image acquisition opening 33.

Furthermore, as the weighting index parameter, the following parameters are usable.

(a) That is, an image acquisition distance that is a distance D between the image acquisition position P and the position of the image acquisition opening 33 can be used as the weighting index parameter. For example, as shown in FIG. 8A and FIG. 8B, when the distance D between the image acquisition opening 33 and the image acquisition position P is near and is in a distance range $d_1$ in which the surface of the image acquisition position can be observed in detail, the weighting information is enlarged. Conversely, when the distance D is far and is in a distance range $d_2$ in which the surface of the image acquisition position cannot be observed in detail, the weighting information is made smaller in proportion to the distance D. As described above, when the image acquisition distance that is the distance D between the image acquisition position P and the position of the image acquisition opening 33 is used as the weighting index parameter, the display calculating section 64 needs to include a function of calculating the image acquisition distance.

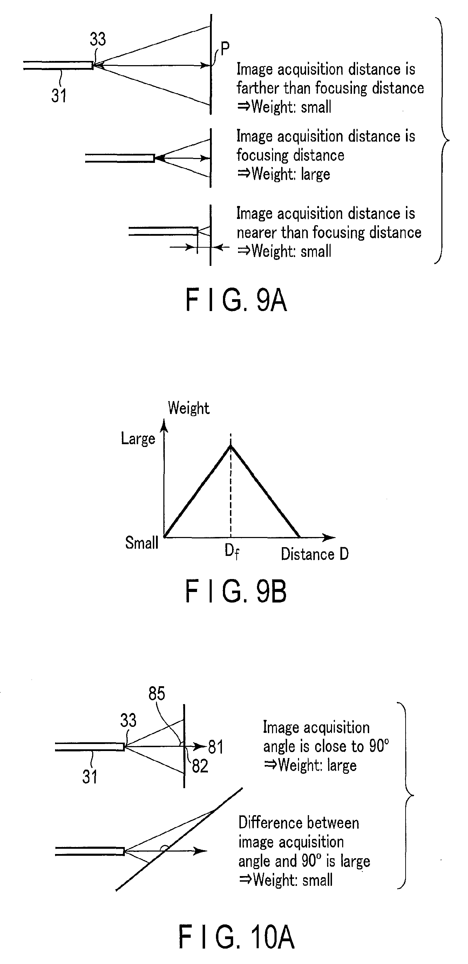

Furthermore, concerning the distance D between the image acquisition opening 33 and the image acquisition position P, another way to attach weighting information may be as shown in FIG. 9A and FIG. 9B. That is, the weighting information is maximized at a distance $D_f$ where the image acquisition section 34 is focused, and the weighting information is made smaller when the distance D is far or near from the focusing distance $D_f$. In this case, an image (the acquired image) acquired at the focusing distance $D_f$ is easily recognized by the operator, and hence the weighting information is enlarged.

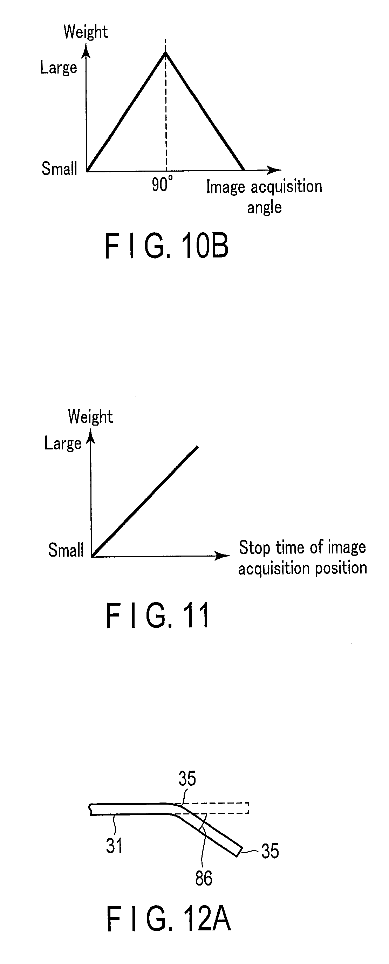

(b) Alternatively, as shown in FIG. 10A, an image acquisition angle 85 formed by an image acquisition direction 81 that is a direction from the image acquisition opening 33 to a center of an image acquisition region 83 in which image is acquired by the image acquisition section 34 and a plane of the image acquisition position P may be used as the weighting index parameter. In this case, as shown in FIG. 10B, when the image acquisition angle 85 is close to 90°, the image is easily acquired and hence the weighting information is large, and an obliquely seen image in which the image acquisition angle 85 is close to 0° is hard to be observed and hence the weighting information is small. As described above, when the image acquisition angle 85 is used as the weighting index parameter, the display calculating section 64 needs to include a function of calculating the image acquisition angle.

(c) In addition, a stop time of the position of the image acquisition section 34 in relation to the insertion subject 2 or a stop time of the image acquisition position may be used as the weighting index parameter. In this case, it is considered that the stop time is an observation time, and as shown in FIG. 11, the weighting information is large in a region where the observation time is long, and the weighting information is small in a region where the observation time is short. As described above, when the stop time is used as the weighting index parameter, the display calculating section 64 needs to include a function of calculating the stop time.

(d) It is to be noted that the speed of the image acquisition position or the speed of the image acquisition opening 33 in relation to the insertion subject 2 may be a movement amount of the image acquisition position or the position of the image acquisition opening 33 in relation to the insertion subject 2 in an exposure time of the image acquisition section 34. When the movement amount in the exposure time is large, a blurring amount of the image is large and hence the weighting information is made smaller, and when the movement amount is small, the blurring amount is small and hence the weighting information is enlarged. In this case, the display calculating section 64 needs to include a function of calculating the exposure time.

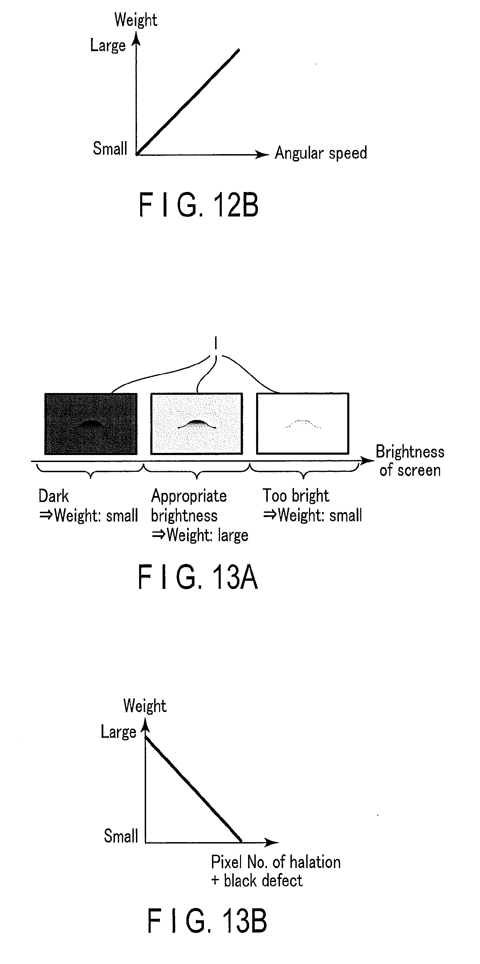

(e) In addition, a temporal change of a bend amount of the bending portion 35 may be used as the weighting index parameter. That is, as shown in FIG. 12A, when an angle formed by one longitudinal direction of the inserting section 31 and the other longitudinal direction of the inserting section 31 via the bending portion 35 is a bend amount 86, the temporal change of the bend amount 86 means an angular speed thereof. As shown in FIG. 12B, when the angular speed is fast, the operator does not easily recognize the image and hence the weighting information is made smaller, and when the angular speed is slow, the operator easily recognizes the image and hence the weighting information is enlarged. In this case, the display calculating section 64 needs to include a function of calculating the angular speed.

Furthermore, the weighting index parameter may be based on the image acquired by the image acquisition section 34. As the weighting index parameter in this case, the following parameters are usable.

(a) That is, as shown in FIG. 13A, a brightness of an image I acquired by the image acquisition section 34 can be used as the weighting index parameter. For example, when there are many ranges in which halation or a black defect occurs in the image, it can be judged that the observation cannot be done. Therefore, as shown in FIG. 13B, the weighting information is made smaller in proportion to a sum of the number of pixels having the halation and the number of pixels having the black defects. In this case, the display calculating section 64 needs to include a function of calculating the brightness of the image acquired by the image acquisition section 34.

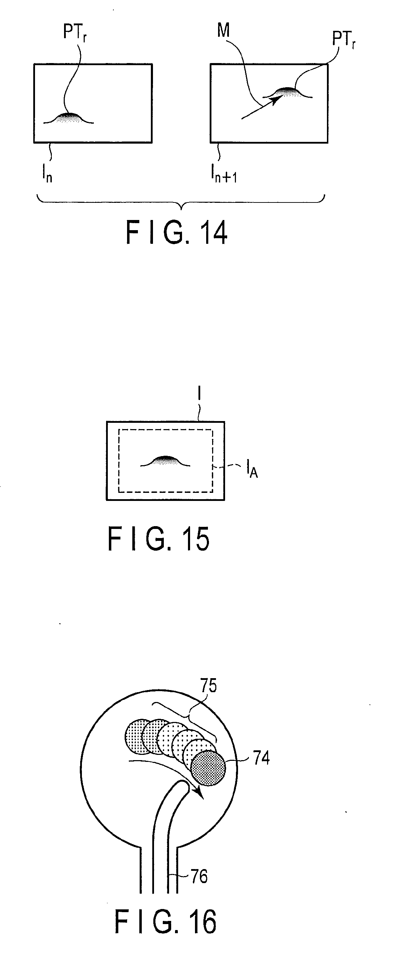

(b) In addition, the blurring amount of the image may be used as the weighting index parameter. That is, as shown in FIG. 14, the display calculating section 64 obtains a movement amount M of a pattern $PT_r$ of interest on an image as a blurring amount between an image It acquired at a time $t_n$ and an image $I_{t+1}$ acquired at a time $t_{n+1}$ by pattern recognition. When the movement amount M is large, the blurring amount is large and the image is hard to be recognized, so that the weighting information is made smaller. Conversely, when the movement amount M is small, the blurring amount is small and the image is easily recognized, so that the weighting information enlarges. In this case, the display calculating section 64 needs to include a function of calculating the blurring amount of the image by pattern matching of the images acquired at different image acquisition times.

In addition, when the image is used as the weighting index parameter, the whole image is not used, but a limited predetermined range in the image may be used as the weighting index parameter. For example, a region of interest in the insertion subject 2 is usually caught at a center of the image, and hence the center of the image is often more important than a periphery thereof. Therefore, as shown in FIG. 15, a range that is vertically and horizontally 80% from the center of the image I is set to a range IA that becomes an object of the weighting index parameter, and a brightness of the range IA is used as the weighting index parameter. As described above, the range in which the display calculating section 64 calculates the weighting information can be set to a predetermined range of the image acquired by the image acquisition section 34, e.g., a region including the center of the image.

On the other hand, when the insertion subject 2 is tubular, the center of the image includes an inner part of a tube and cannot be often observed, and hence the range is limited to the periphery of the image. This can be realized by, for example, setting the predetermined range of the image in which the display calculating section 64 calculates the weighting information to a range of the image in which a distance between the image acquisition opening 33 and the insertion subject 2 in an image acquisition range 87 (see FIG. 1A) that is a range in which the image acquisition section 34 performs the image acquisition is a predetermined distance or less.

In addition, as the display format on the basis of the weighting information, it has been described that the change of the display of the image acquisition position is the change of the depth of the color, but the change may be a change to another color or a change of transparency. Alternatively, a set of points may be displayed, and the change may be a change of a density of the points as shown in FIG. 16.

In addition, it has been described that the inserting section 31 is the flexible tubular member, but the inserting section may have an inflexible. When the inserting section 31 has the inflexible in this manner, the fiber shape sensor 4 is not required, and the insertion and rotation detecting section 5 detects the position of the inserting section distal end in relation to the insertion subject 2. It is to be noted that the direction of the inserting section distal end can be obtained on the basis of, for example, a movement history of the image acquisition region 83 which is detected from the acquired image by the pattern recognition or the like.

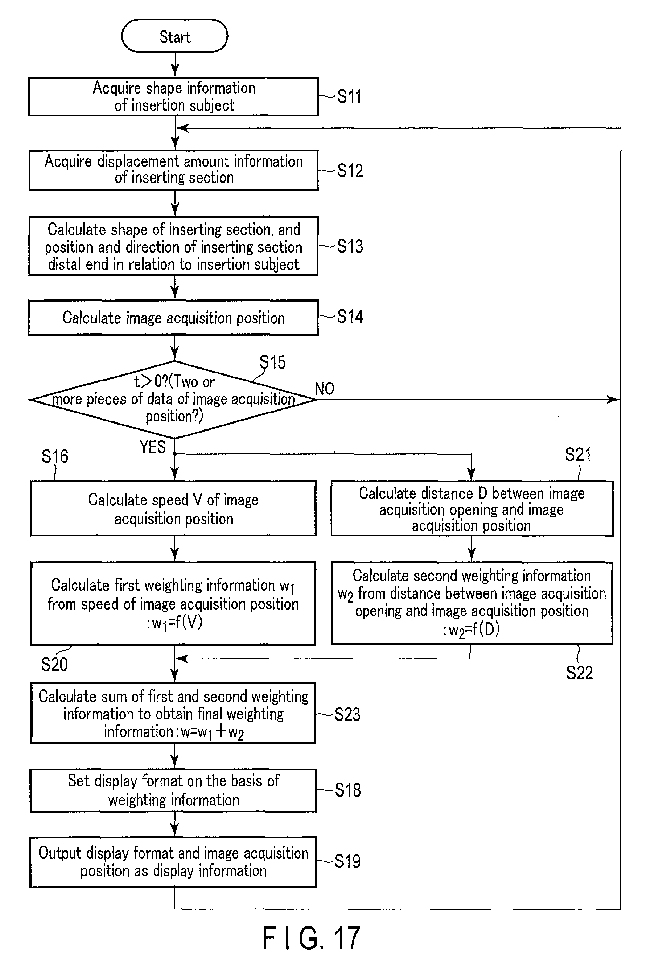

In addition, the description has been given as to the example where one weighting index parameter is used, but the weighting index parameters may be set and the weighting index parameters may be calculated. For example, when the first weighting index parameter is the speed of the image acquisition position and the second weighting index parameter is the distance between the image acquisition opening 33 and the image acquisition position, an operation of the observation supporting device 6 is as shown in FIG. 17.

That is, the above operation of the step S11 to the step S16 as described with reference to FIG. 5A is performed to calculate the speed V of the image acquisition position, and then the display calculating section 64 calculates first weighting information $w_1$ from this obtained speed V (step S20). For example, the weighting is performed in accordance with a relation (the following equation (5)) in which the weighting information becomes smaller in proportion to the speed V of the image acquisition position as shown in FIG. 5B.

$$w_1 = f(V) \tag{5}$$

Additionally, in parallel with this calculation, the display calculating section 64 calculates the distance D between the image acquisition position P and the position of the image acquisition opening 33 (step S21). It is to be noted that when the speed V of the image acquisition position is obtained in the above step S16, the current acquired image and one previous acquired image are used, but this distance D is a distance at a time when the current image is acquired. Furthermore, the display calculating section 64 calculates second weighting information $w_2$ from this obtained distance D (step S22). For example, the weighting is performed in accordance with a relation (the following equation (6)) in which, as shown in FIG. 8B, when the distance D is in the distance range $d_1$, the weighting information is large, and when the distance D is in the distance range $d_2$, the weighting information becomes smaller in proportion to the distance D.

$$w_2 = f(D) \tag{6}$$

Furthermore, as represented by the following equation (7), a sum of the first weighting information $w_1$ and the second weighting information $w_2$ is calculated (a product may be calculated or another calculating method may be used) to obtain final weighting information w.

$$w = w_1 + w_2 \tag{7}$$

Afterward, the processing advances to the abovementioned step S18, in which the display calculating section 64 sets the display format on the basis of the obtained final weighting information w.

When the weighting is performed from the weighting index parameters in this manner, an accuracy of the importance of the image acquisition position information enhances.

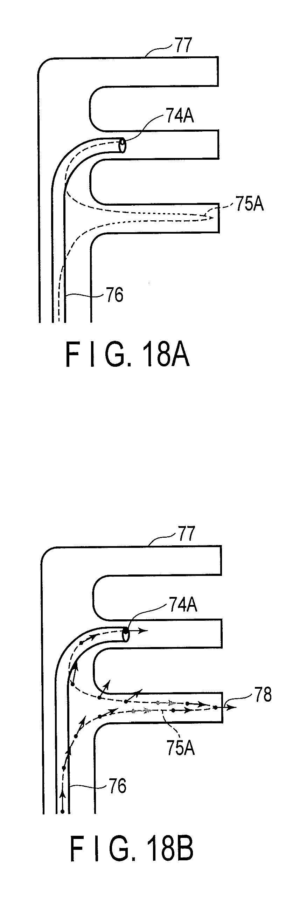

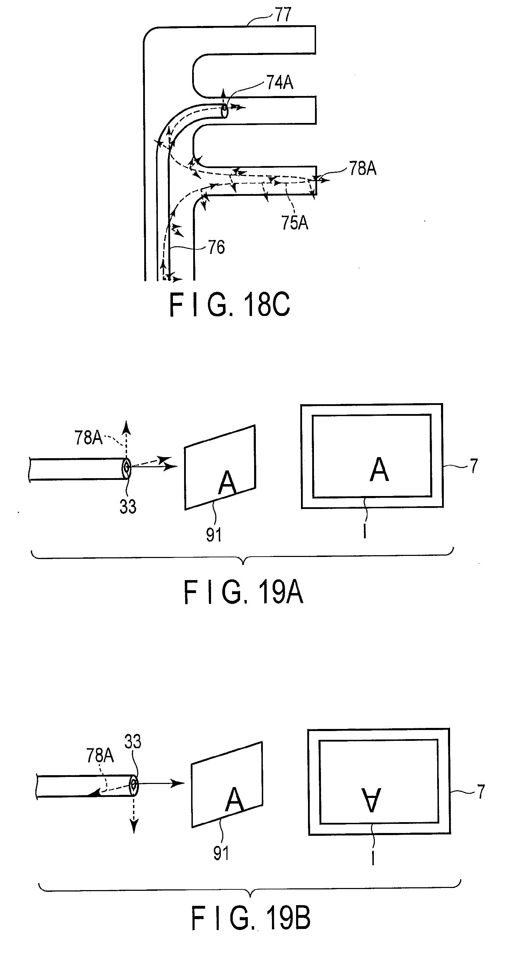

The description has been given as to the example where the history of the image acquisition position information is displayed as the position locus display 75, but further, the history of the position of the inserting section distal end, e.g., the image acquisition opening 33 may be displayed. This fact will be described with reference to FIG. 18A to FIG. 18C and FIG. 19A and FIG. 19B. FIG. 18A to FIG. 18C show an example of the display in the display device 7 when the inserting section 31 is inserted into a branched piping line as the insertion subject 2.

In FIG. 18A, the inserting section shape schematic display 76 showing the shape of the inserting section 31, a current position display 74A showing the current position of the image acquisition opening 33 and a position locus display 75A showing the locus of the position of the image acquisition opening 33 are displayed on a two-dimensional view 77 showing the shape of the insertion subject. Additionally, the position locus display 75 that is the locus of the image acquisition position is omitted. From the locus display of a distal position, there are seen the position of the insertion subject 2 which is passed by the image acquisition opening 33 and the position at a current time. In addition, the position locus display 75A may be displayed in a display format set on the basis of the weighting information. As the display format in this case, for example, a color, a type, a thickness or presence/absence of a broken line can be changed.

When the position of the image acquisition opening 33 is recognized, a specific position of the image acquisition object which is reached is recognized. When the current position is exactly recognized, the observation or treatment to be carried out at the current position or investigation of a path from the current position to a target position can be performed by using this information, without presuming that the current position would be this place. Therefore, it is not necessary to repeat trial and error in reaching the target position, nor is it necessary to confirm whether or not the target position was reached, by various methods including, for example, a method of observing the acquired image. As a result, there is a high possibility that the target position can be reached at one time by taking the path close to the shortest course from the current position to the target position, so that time can be reduced and furthermore, a situation concerning the position can be grasped, which leads to a calmed and assured operation.

Furthermore, in addition to the history of the position of the image acquisition opening 33, a history of a one-dimensional direction in which the image acquisition opening 33 is directed may be displayed. The direction in which the image acquisition opening 33 is directed is, for example, the center of the viewing field (the image acquisition region 83). FIG. 18B shows the direction in which the image acquisition opening 33 is directed by an arrow 78. In addition to the current position and direction of the image acquisition opening 33, information of directions at several positions on the locus of the image acquisition opening 33 is added by using the arrows 78. From the display of the locus and direction of the image acquisition opening 33, it is possible to recognize the locus of the distal position which is the position information of the image acquisition opening 33 at the inserting section distal end, and a specific direction in which the image acquisition opening is directed while the position of the image acquisition opening changes. At this time, for example, the color, type, thickness or presence/absence of the arrow 78 may be changed on the basis of the weighting information. Furthermore, information of the position and direction of the image acquisition opening 33 may be combined with the image acquisition position information. In consequence, the position and direction of the image acquisition opening 33 when the image is acquired are seen in relation to the image acquisition position information, i.e., the position at which image is acquired in the past.

It is to be noted that depending on the optical system for the image acquisition, in the present example, the direction in which the image acquisition opening 33 present at the inserting section distal end is directed is the center of the viewing field and is the middle of the acquired image.

When the position and direction of the inserting section distal end are recognized, a position reached and a direction in the image acquisition object are recognized. An observation viewing field direction and the viewing field center are seen from the current position and direction. When the reaching position and direction or the observation viewing field direction and viewing field center are exactly recognized, it is possible to perform the observation or treatment to be carried out in accordance with the current position and direction, or the investigation of the path from the current position to the target position and the shape or operating method of the inserting section 31 during the movement, by use of this information without presuming that the current position and direction would be such the position and direction. In particular, when the direction of the inserting section distal end is recognized, it is possible to investigate an operating method or procedure such as insertion/extraction or bending for the purpose of reaching the target position or direction.

The history of the direction in which the image acquisition opening 33 is directed may three-dimensionally be shown to indicate the direction including a posture or rotation of the inserting section distal end. As shown in FIG. 19A and FIG. 19B, even in a case where the direction in which the image acquisition opening 33 is directed is the same, when the inserting section 31 rotates, the image acquisition opening 33 to an image acquisition object 91 also rotates. In FIG. 19A and FIG. 19B, the image acquisition opening rotates as much as 180°, and hence the upside and downside are reversed, and in this case, the image I acquired by the image acquisition section 34 is also displayed upside down.

In FIG. 18C, when the rotation of a coordinate system fixed to the inserting section distal end, i.e., the coordinate system in which the position and a posture of the inserting section distal end does not change is defined as "a three-dimensional direction" of the inserting section distal end, a direction in which the image acquisition opening 33 is directed is shown by arrows 78A of three directions (an x-direction, a y-direction, and a z-direction) to show the three-dimensional direction (the posture) of the inserting section distal end. At this time, for example, a color, a type, a thickness or presence/absence of the arrows 78A may be changed on the basis of the weighting information. When the position and three-dimensional direction of the inserting section distal end are recognized in this manner, for example, the image acquisition direction including the rotation of the inserting section distal end at the image acquisition position is recognized. In addition, an influence of the rotation in the distal end direction can be taken into consideration during the treatment or the like other than the image acquisition. In addition, when the history of the three-dimensional direction is displayed, a direction including the rotation of the image acquisition opening 33 when the image is acquired is seen in relation to the image acquisition position information, i.e., the position at which image is acquired in the past.

[Second Embodiment]

A second embodiment of the present invention is different from the above first embodiment in the following respects. That is, an observation supporting device 6 concerned with the present second embodiment sets a threshold value to a weighting index parameter and determines weighting information of an image acquisition position by comparison with the threshold value.

Hereinafter, a part different from the above first embodiment will only be described.

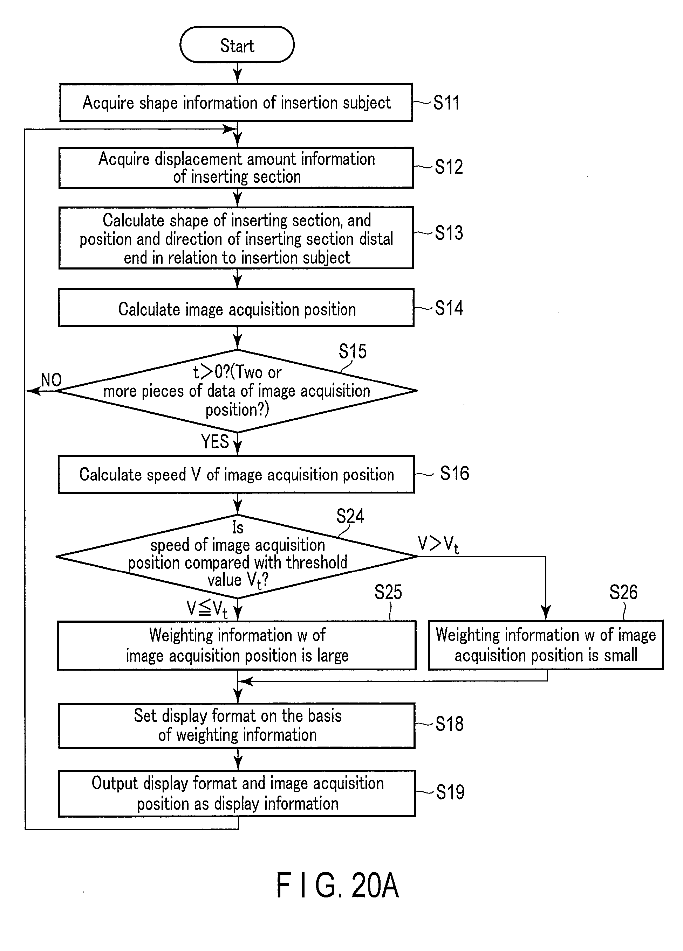

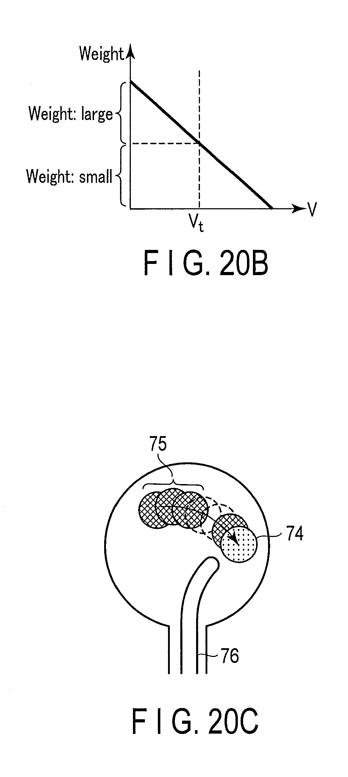

FIG. 20A corresponds to FIG. 5A in the above first embodiment. Similarly to the first embodiment, the above operation of the step S11 to the step S16 is performed to calculate a speed V of the image acquisition position. Afterward, a display calculating section 64 compares the speed V of the image acquisition position with a threshold value $V_t$ beforehand stored in the display calculating section 64 (step S24), and determines weighting information w from the comparison result as shown in FIG. 20B. That is, when the speed V of the image acquisition position is the threshold value $V_t$ or less, the display calculating section 64 determines that the weighting information w is large (step S25), and when the speed V is larger than the threshold value $V_t$, the section determines that the weighting information w is small (step S26). It is to be noted that here, the threshold value $V_t$ is, for example, the maximum speed of the image acquisition position at which a person (an operator) can recognize an image.

Afterward, similarly to the above first embodiment, the processing advances to the above step S18, in which the display calculating section 64 sets a display format on the basis of the obtained final weighting information w.

It is to be noted that as the display format, similarly to the above first embodiment, a change of a color, a change of a transparency or a change of a density of points may be used, but when information of the image acquisition position is divided into two types of weighting information by the comparison with the threshold value as in the present embodiment, as shown in FIG. 20C, presence/absence of an image acquisition position display may be used. That is, there is used the display format in which the display is performed as a position locus display 75 concerning the image acquisition position where the weighting information w is large, but the position locus display 75 is not performed at the image acquisition position where the weighting information w is small (in FIG. 20C, broken lines are shown for explanation). It is to be noted that a current position display 74 is performed irrespective of a size of the weighting information w.

As described above, according to the present second embodiment, a weighting index parameter such as the speed V of the image acquisition position is compared with the threshold value (e.g., $V_t$) to calculate the weighting information, so that the information of the image acquisition position can be divided into the two types of weighting information. Therefore, for example, it can be seen whether the information is image acquisition position information when the speed is faster than that of the threshold value or image acquisition position information when the speed is slower.

In addition, the threshold value $V_t$ of the speed of the image acquisition position is set to the maximum speed of the image acquisition position where the person (the operator) can recognize the image, so that it is seen that the speed is in a range in which an image acquisition section 34 performs the image acquisition but the person cannot recognize, i.e., cannot observe.

Furthermore, as to the image acquisition position, on the basis of the weighting information of the image acquisition position, a locus of the image acquisition position is displayed when the weighting information is large, i.e., the speed V of the image acquisition position is slower than the threshold value $V_t$, and the locus of the image acquisition position is not displayed when the weighting information is small, i.e., the speed V of the image acquisition position is faster than the threshold value $V_t$. In consequence, a locus of the range in which the movement of the image acquisition position is so fast that the operator cannot observe is not displayed as an observed range.

It is to be noted that the threshold value is not limited to one value, and the threshold values may be used.

In addition, the weighting index parameter is not limited to the speed of the image acquisition position, and needless to say, various parameters can be applied as described in the above first embodiment.

For example, when the weighting index parameter is a distance between an inserting section distal end and an insertion subject 2, the threshold value can be a range of a subject field depth. When the weighting index parameter is a brightness of the image, the threshold value can be presence/absence of halation and black defects. Furthermore, as the threshold value, the operator may input any value.

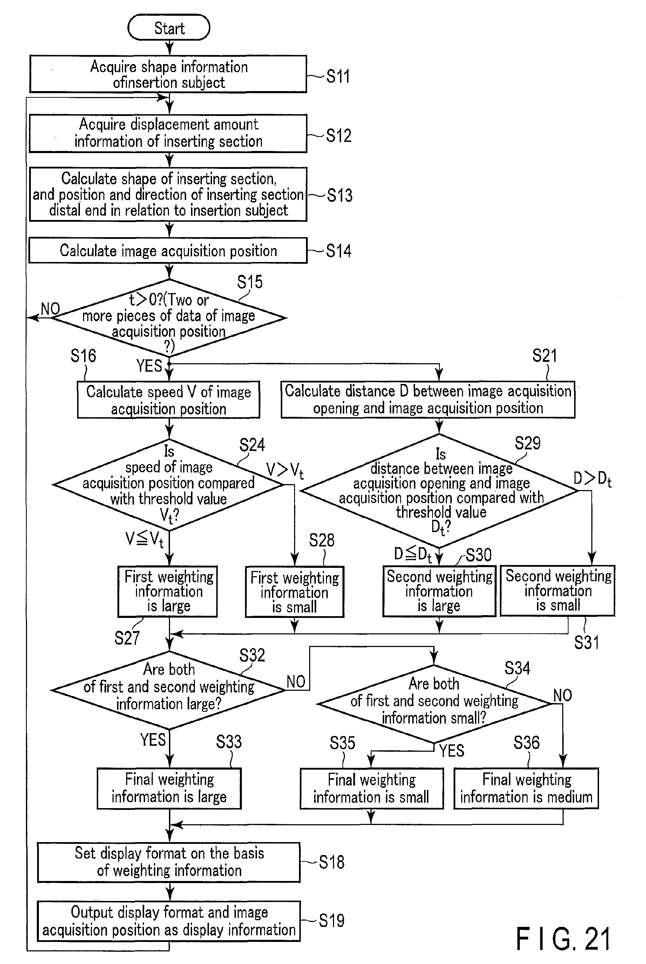

In addition, the description has been given as to the example where one weighting index parameter is used, but the weighting index parameters may be set and the weighting index parameters may be calculated. For example, when a first weighting index parameter is the speed of the image acquisition position and a second weighting index parameter is a distance between an image acquisition opening 33 and the image acquisition position, an operation of the observation supporting device 6 is as shown in FIG. 21.

That is, the above operation of the step S11 to the step S16 as described with reference to FIG. 5A is performed to calculate the speed V of the image acquisition position, and then the display calculating section 64 compares the speed V of the image acquisition position with the threshold value $V_t$ beforehand stored in the display calculating section 64 (step S24), and determines first weighting information from the comparison result. That is, when the speed V of the image acquisition position is the threshold value $V_t$ or less, the display calculating section 64 determines that the first weighting information is large (step S27), and when the speed V is larger than the threshold value $V_t$, the section determines that the first weighting information is small (step S28).

Additionally, in parallel with this determination, the display calculating section 64 calculates a distance D between the image acquisition position P and a position of the image acquisition opening 33 (step S21). Furthermore, the display calculating section 64 compares the distance D with a threshold value $D_t$ beforehand stored in the display calculating section 64 (step S29), and determines second weighting information from the comparison result. That is, when the distance D between the image acquisition position P and the position of the image acquisition opening 33 is the threshold value $D_t$ or less, the display calculating section 64 determines that the second weighting information is large (step S30), and when the distance D is larger than the threshold value $D_t$, the section determines that the second weighting information is small (step S31).

When the first and second weighting index parameters are compared with the threshold values in this manner, respectively, to determine the size of the first and second weighting information, the display calculating section 64 next judges whether or not both of the first and second weighting information are large (step S32). When both of the first and second weighting information are large, the display calculating section 64 determines that the final weighting information is large (step S33).

On the other hand, when both of the first and second weighting information are not large, the display calculating section 64 further judges whether or not both of the first and second weighting information are small (step S34). When both of the first and second weighting information are small, the display calculating section 64 determines that the final weighting information is small (step S35).

In addition, when both of the first and second weighting information are not small, i.e., when one information is large and the other information is small, the display calculating section 64 determines that the final weighting information is medium (step S36).

In consequence, the display calculating section 64 determines the final weighting information from three stages of weighting information. Afterward, the step advances to the abovementioned step S18, in which the display calculating section 64 sets the display format on the basis of the obtained final weighting information w.

The weighting is performed from the weighting index parameters in this manner, so that an accuracy of importance of the image acquisition position information enhances.

The present invention has been described above on the basis of the embodiments, but needless to say, the present invention is not restricted to the abovementioned embodiments and various modifications or applications are possible within the gist of the present invention.

For example, a program of software to realize the function shown in the flowchart of FIG. 5A, FIG. 17, FIG. 20A or FIG. 21 is supplied to a computer, and the computer executes this program to enable realization of the above function of the observation supporting device 6.


**Claims**

1. An observation apparatus **characterized by** comprising:

 an inserting section that is to be inserted into an insertion subject and includes an image acquisition opening;
 an image acquisition section that receives light entering into the image acquisition opening and acquires image;

a relative position detecting section that detects a relative position, in relation to the insertion subject, of a portion of the inserting section which becomes a position detection object;

an insertion subject shape acquiring section that acquires shape information of the insertion subject;

an image acquisition position calculating section that calculates an image acquisition position that is at least one of an image acquisition region as a region of the insertion subject which is being acquired image by the image acquisition section, a part of the image acquisition region and a point in the image acquisition region, by use of the relative position and the shape information of the insertion subject;

a display calculating section that calculates weighting information of the image acquisition position on the basis of a weighting index parameter, and sets a display format on the basis of the weighting information; and

an output section that outputs the display format and the image acquisition position as display information.

2. The observation apparatus according to claim 1, **characterized in that** the display calculating section sets the display format as at least one of presence/absence of an image acquisition position display, a change of a color, a change of a transparency and a change of a density of points, on the basis of the weighting information.

3. The observation apparatus according to claim 1 or 2, **characterized in that** the display calculating section includes the weighting index parameters, and calculates the weighting information by use of the weighting index parameters.

4. The observation apparatus according to any one of claims 1 to 3, **characterized in that**
the relative position detecting section includes at least one of:

a fiber shape sensor that is disposed in at least the inserting section, and detects a bend amount of the inserting section by use of the fact that optical characteristics of the light guided through an optical fiber change in accordance with a bend;

an insertion amount detecting section that detects an amount of an inserting direction of the inserting section in relation to the insertion subject; and

a rotation amount detecting section that detects an amount of a rotating direction of the inserting section in relation to the insertion subject.

5. The observation apparatus according to any one of claims 1 to 4, **characterized in that** the weighting index parameter is set on the basis of a position of the image acquisition opening in relation to the insertion subject.

6. The observation apparatus according to claim 5, **characterized in that**
the display calculating section further includes a function of calculating an image acquisition distance that is a distance between the image acquisition position and the position of the image acquisition opening, and
the weighting index parameter is the image acquisition distance.

7. The observation apparatus according to claim 5, **characterized in that**
the display calculating section further includes a function of calculating an image acquisition angle that is an angle formed by an image acquisition direction as a direction from the image acquisition opening to a center of the image acquisition region and a plane of the image acquisition position, and
the weighting index parameter is the image acquisition angle.

8. The observation apparatus according to any one of claims 1 to 4, **characterized in that** the weighting index parameter is set on the basis of a temporal change of the image acquisition position or a temporal change of the position of the image acquisition opening in relation to the insertion subject.

9. The observation apparatus according to claim 8, **characterized in that**
the display calculating section further includes a function of calculating a stop time that is a time when the image acquisition position or the position of the image acquisition opening in relation to the insertion subject is stopped, and
the weighting index parameter is the stop time.

10. The observation apparatus according to claim 8, **characterized in that**
the display calculating section further includes a function of calculating a change speed that is a speed at which the image acquisition position or the position of the image acquisition opening in relation to the insertion subject changes, and
the weighting index parameter is the change speed.

**11.** The observation apparatus according to claim 10, **characterized in that**
the display calculating section further includes a function of calculating an exposure time of the image acquisition section, and
the change speed is a movement amount of the image acquisition position at the exposure time or a movement amount of the position of the image acquisition opening in relation to the insertion subject.

**12.** The observation apparatus according to claim 8, **characterized in that**
the inserting section includes a bending portion,
the display calculating section further includes a function of calculating a temporal change of a bend amount that is an angle formed by one longitudinal direction of inserting section and the other longitudinal direction of the inserting section via the bending portion, and
the weighting index parameter is the temporal change of the bend amount.

**13.** The observation apparatus according to any one of claims 1 to 4, **characterized in that** the weighting index parameter is set on the basis of the image acquired by the image acquisition section.

**14.** The observation apparatus according to claim 13, **characterized in that**
the display calculating section further includes a function of calculating a brightness of the image acquired by the image acquisition section, and
the weighting index parameter is the brightness of the image.

**15.** The observation apparatus according to claim 13, **characterized in that**
the display calculating section further includes a function of calculating a blurring amount of the image, and
the weighting index parameter is the blurring amount of the image.

**16.** The observation apparatus according to claim 15, **characterized in that** the display calculating section calculates the blurring amount of the image by pattern matching of the images acquired at different image acquisition times.

**17.** The observation apparatus according to claim 13, **characterized in that** the display calculating section calculates the weighting information in a predetermined range of the image acquired by the image acquisition section.

**18.** The observation apparatus according to claim 17, **characterized in that** the predetermined range of the image is a range of the image in which a distance between the insertion subject and the image acquisition opening in an image acquisition range that is a range in which the image acquisition section acquires the image is a predetermined distance or less.

**19.** The observation apparatus according to claim 17, **characterized in that** the predetermined range of the image is a region including a center of the image.

**20.** The observation apparatus according to any one of claims 1 to 5, 8 and 13, **characterized in that** the display calculating section further compares the weighting index parameter with a threshold value to calculate the weighting information.

**21.** The observation apparatus according to claim 20, **characterized in that** the threshold value is determined on the basis of at least one of a range of a subject field depth of the image acquisition opening, a speed of image recognition of a person, a value input by an operator, and presence/absence of halation and black defects of the image acquired by the image acquisition section.

**22.** The observation apparatus according to claim 1, **characterized in that** comprising a display device that is input the display information from the output section and displays the display information.

**23.** An observation supporting device for use in an observation apparatus in which an inserting section is inserted into an insertion subject to acquire image of the inside of the insertion subject, the observation supporting device **characterized by** comprising:

a relative position information acquiring section that acquires relative position information, in relation to the insertion subject, of a portion of the inserting section which becomes a position detection object, on the basis of displacement amount information of the inserting section;

an insertion subject shape acquiring section that acquires shape information of the insertion subject;

an image acquisition position calculating section that calculates an image acquisition position that is at least one of an image acquisition region as a region of the insertion subject which is being acquired image by the observation apparatus, a part of the image acquisition region and a point in the image acquisition region, by use of the relative position information and the shape information of the insertion subject;

a display calculating section that calculates weighting information of the image acquisition position on the basis of a weighting index parameter, and sets a display format on the basis of the weighting information; and

an output section that outputs the display format and the image acquisition position as display information.

24. An observation supporting method for use in an observation apparatus in which an inserting section is inserted into an insertion subject to acquire image of the inside of the insertion subject, the observation supporting method **characterized by** comprising:

a position information acquiring step of acquiring relative position information, in relation to the insertion subject, of a position of the inserting section which becomes a detection object, on the basis of displacement amount information of the inserting section;

an insertion subject shape acquiring step of acquiring shape information of the insertion subject;

an image acquisition position calculating step of calculating an image acquisition position that is at least one of an image acquisition region as a region of the insertion subject which is being acquired image by the observation apparatus, a part of the image acquisition region and a point in the image acquisition region, by use of the relative position information and the shape information of the insertion subject;

a display calculating step of calculating weighting information of the image acquisition position on the basis of a weighting index parameter, and setting a display format on the basis of the weighting information; and

an output step of outputting the display format and the image acquisition position as display information.

25. A program which allows a computer to execute:

a position information acquiring procedure of acquiring relative position information, in relation to an insertion subject, of a position of an inserting section which becomes a detection object, on the basis of displacement amount information of the inserting section in an observation apparatus in which the inserting section is inserted into the insertion subject to acquire image of the inside of the insertion subject;

an insertion subject shape acquiring procedure of acquiring shape information of the insertion subject;

an image acquisition position calculating procedure of calculating an image acquisition position that is at least one of an image acquisition region as a region of the insertion subject which is being acquired image by the observation apparatus, a part of the image acquisition region and a point in the image acquisition region, by use of the relative position information and the shape information of the insertion subject;

a display calculating procedure of calculating weighting information of the image acquisition position on the basis of a weighting index parameter, and setting a display format on the basis of the weighting information; and

an output procedure of outputting the display format and the image acquisition position as display information.

FIG. 1A

F I G. 1B

F I G. 2A

Light transmission quantity: large

F I G. 2B

Light transmission quantity: medium

Light transmission quantity: small

F I G. 2C

F I G. 3

Insertion amount,
rotation amount

Displacement
amount
calculating
portion ~55

~5

54

51

53 52

31

Insertion
amount

Rotation
amount

F I G. 4A

54

Rotating
direction

31

y

x

Inserting
direction

~α'

α

PT$_n$

PT$_{n+1}$

F I G. 4B

Start

Acquire shape information of insertion subject — S11

Acquire displacement amount information
of inserting section — S12

Calculate shape of inserting section, and position
and direction of inserting section distal
end in relation to insertion subject — S13

Calculate image acquisition position — S14

t>0?
(Two or more pieces of
data of image acquisition position
?) — S15

NO →

YES ↓

Calculate speed V of image acquisition position — S16

Calculate weighting information w of image
acquisition position from speed of image
acquisition position: w=f(V) — S17

Set display format on the basis
of weighting information — S18

Output display format and image acquisition
position as display information — S19

F I G. 5A

Weight

Large

Small

Speed V of image
acquisition position

F I G. 5B

Display color of image
acquisition position

Deep

Light

Small          Large

Weight

## F I G. 6A

75

74

76

## F I G. 6B

Y-axis

Y-Z plane

2

Z-axis          X-axis

## F I G. 7A

Y-axis

Y-Z plane

2

Z-axis

X-axis

F I G. 7B

33

P

31

Image acquisition
distance is near
⇒Weight: large

Image acquisition
distance is far
⇒Weight: small

F I G. 8A

Weight

Large

Small

$d_1$          $d_2$          Distance D

F I G. 8B

33

31

P

Image acquisition distance is
farther than focusing distance
⇒Weight: small

Image acquisition distance is
focusing distance
⇒Weight: large

Image acquisition distance is
nearer than focusing distance
⇒Weight: small

F I G. 9A

Weight

Large

Small

$D_f$

Distance D

F I G. 9B

85

33

81

82

31

Image acquisition
angle is close to 90°
⇒Weight: large

Difference between
image acquisition
angle and 90° is large
⇒Weight: small

F I G. 10A

F I G. 10B

F I G. 11

F I G. 12A

F I G. 12B

Dark
⇒Weight: small

Appropriate brightness
⇒Weight: large

Too bright
⇒Weight: small

F I G. 13A

F I G. 13B

PT$_r$                M        PT$_r$

I$_n$          I$_{n+1}$

F I G. 14

I

I$_A$

F I G. 15

75

74

76

F I G. 16

Start

Acquire shape information
of insertion subject ─S11

Acquire displacement amount information
of inserting section ─S12

Calculate shape of inserting section, and
position and direction of inserting section ─S13
distal end in relation to insertion subject

Calculate image acquisition position ─S14

S15

t>0?(Two or
more pieces of data of image acquisition
position?)

NO

YES

S16

Calculate speed V of image
acquisition position

S21

Calculate distance D between image
acquisition opening and image
acquisition position

Calculate first weighting information $w_1$
from speed of image acquisition position
: $w_1 = f(V)$

Calculate second weighting information
$w_2$ from distance between image acquisition
opening and image acquisition position
: $w_2 = f(D)$

S20

S22

Calculate sum of first and second weighting
information to obtain final weighting ─S23
information: $w = w_1 + w_2$

Set display format on the basis of
weighting information ─S18

Output display format and image acquisition
position as display information ─S19

F I G. 17

F I G. 18A

F I G. 18B

F I G. 18C

F I G. 19A

F I G. 19B

Start

Acquire shape information of insertion subject — S11

Acquire displacement amount information of inserting section — S12

Calculate shape of inserting section, and position and direction of inserting section distal end in relation to insertion subject — S13

Calculate image acquisition position — S14

S15
t>0?(Two or more pieces of data of image acquisition position?)

NO

YES

Calculate speed V of image acquisition position — S16

S24
Is speed of image acquisition position compared with threshold value $V_t$?

$V>V_t$

$V \leqq V_t$

S25
Weighting information w of image acquisition position is large

S26
Weighting information w of image acquisition position is small

Set display format on the basis of weighting information — S18

Output display format and image acquisition position as display information — S19

F I G. 20A

F I G. 20B

F I G. 20C

Start

Acquire shape information of insertion subject — S11

Acquire displacement amount information of inserting section — S12

Calculate shape of inserting section, and position and direction of inserting section distal end in relation to insertion subject — S13

Calculate image acquisition position — S14

S15

t>0?(Two or more pieces of data of image acquisition position ?) — NO

YES

S16

Calculate speed V of image acquisition position

S21

Calculate distance D between image acquisition opening and image acquisition position

S24

Is speed of image acquisition position compared with threshold value $V_t$? — $V>V_t$

$V≤V_t$

S29

Is distance between image acquisition opening and image acquisition position compared with threshold value $D_t$? — $D>D_t$

$D≤D_t$ — S30

First weighting information is large

First weighting information is small — S28

Second weighting information is large

Second weighting information is small

S27

S31

S32

Are both of first and second weighting information large? — NO

YES — S33

S34

Are both of first and second weighting information small? — NO

S35 — YES — S36

Final weighting information is large

Final weighting information is small

Final weighting information is medium

Set display format on the basis of weighting information — S18

Output display format and image acquisition position as display information — S19

F I G. 21

**INTERNATIONAL SEARCH REPORT**

| International application No. |
| --- |
| PCT/JP2013/077680 |

A. CLASSIFICATION OF SUBJECT MATTER
*A61B1/00*(2006.01)i, *G02B23/24*(2006.01)i

According to International Patent Classification (IPC) or to both national classification and IPC

B. FIELDS SEARCHED

Minimum documentation searched (classification system followed by classification symbols)
A61B1/00, G02B23/24

Documentation searched other than minimum documentation to the extent that such documents are included in the fields searched

| Jitsuyo Shinan Koho | 1922–1996 | Jitsuyo Shinan Toroku Koho | 1996–2013 |
| Kokai Jitsuyo Shinan Koho | 1971–2013 | Toroku Jitsuyo Shinan Koho | 1994–2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 2011-206425 A (Fujifilm Corp.), 20 October 2011 (20.10.2011), entire text; all drawings (Family: none) | 1-23,25 |
| A | JP 2009-125394 A (Toshiba Corp.), 11 June 2009 (11.06.2009), entire text; all drawings (Family: none) | 1-23,25 |
| A | WO 2012/132638 A1 (Olympus Medical Systems Corp.), 04 October 2012 (04.10.2012), entire text; all drawings & JP 5159995 B          & US 2013/0096423 A1 & CN 103068297 A | 1-23,25 |

| ☒ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 13 December, 2013 (13.12.13) | Date of mailing of the international search report 24 December, 2013 (24.12.13) |
| --- | --- |
| Name and mailing address of the ISA/ Japanese Patent Office | Authorized officer |
| Facsimile No. | Telephone No. |

Form PCT/ISA/210 (second sheet) (July 2009)

36

**INTERNATIONAL SEARCH REPORT**

| International application No. |
|---|
| PCT/JP2013/077680 |

C (Continuation). DOCUMENTS CONSIDERED TO BE RELEVANT

| Category* | Citation of document, with indication, where appropriate, of the relevant passages | Relevant to claim No. |
|---|---|---|
| A | JP 2012-191978 A (Fujifilm Corp.),<br>11 October 2012 (11.10.2012),<br>entire text; all drawings<br>& US 2012/0238807 A1 | 1-23,25 |
| A | WO 2012/14438 A1 (Fujifilm Corp.),<br>02 February 2012 (02.02.2012),<br>entire text; all drawings<br>& JP 2012-24518 A | 1-23,25 |

Form PCT/ISA/210 (continuation of second sheet) (July 2009)

## INTERNATIONAL SEARCH REPORT

| International application No. |
| --- |
| PCT/JP2013/077680 |

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 24
   because they relate to subject matter not required to be searched by this Authority, namely:
   Claim 24 pertains to a method for treatment of the human body or animal body by surgery or therapy and thus relates to a subject matter on which this International Searching Authority is not required to carry out an international search under the provisions of PCT Article 17(2)(a)(i) and PCT Rule 39.1(iv).

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.    Consequently, this international search report is restricted to the invention first mentioned in the claims; it is covered by claims Nos.:

**Remark on Protest**    ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

## REFERENCES CITED IN THE DESCRIPTION

**Patent documents cited in the description**

- US 6846286 B